(19) Europäisches Patentamt
European Patent Office
Office européen des brevets

(11) Veröffentlichungsnummer: **0 031 302**
**A2**

(12) # EUROPÄISCHE PATENTANMELDUNG

(21) Anmeldenummer: **80810394.9**

(22) Anmeldetag: **15.12.80**

(51) Int. Cl.³: **C 07 D 249/20**
C 08 K 5/34

(30) Priorität: **21.12.79 CH 11405 79**
**12.05.80 CH 3703/80**

(43) Veröffentlichungstag der Anmeldung:
**01.07.81 Patentblatt 81/26**

(84) Benannte Vertragsstaaten:
**DE FR GB IT**

(71) Anmelder: **CIBA-GEIGY AG**
**Patentabteilung Postfach**
**CH-4002 Basel(CH)**

(72) Erfinder: **Rody, Jean, Dr.**
**Rütiring 82**
**CH-4125 Riehen(CH)**

(72) Erfinder: **Slongo, Mario, Dr.**
**Pappelweg 8**
**CH-4132 Muttenz(CH)**

(54) **Benzotriazol UV-Absorber, Verfahren zu ihrer Herstellung und stabilisierte Mischungen.**

(57) Neue Verbindungen der Formel I

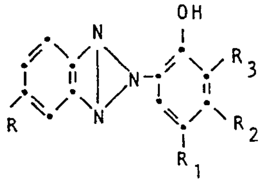

(1),

worin die Symbole die in Anspruch 1 angegebene Bedeutung haben.

Die Verbindungen können als UV-Stabilisatoren, insbesondere in Lacken, verwendet werden.

EP 0 031 302 A2

Croydon Printing Company Ltd.

CIBA-GEIGY AG
Basel (Schweiz)

3-12657/1+2/+

# Benzotriazol UV-Absorber, Verfahren zu ihrer Herstellung und stabilisierte Mischungen

Die vorliegende Erfindung betrifft 2-Aryl-2H-benzotriazole als Lichtschutzmittel für organisches Material, sowie stabilisierte Mischungen.

UV-Absorber vom Typ der o-Hydroxyphenyl-2H-benzotriazole geniessen seit langem ein erhebliches wirtschaftliches Interesse als Lichtstabilisatoren für organisches Material.

Solche Verbindungen, deren Herstellung und Verwendung sind z.B. aus den folgenden Patentschriften bekannt: US 3 004 896, US 3 055 896, US 3 072 585, US 3 074 910, US 3 189 615 und US 3 230 194. Diese Stabilisatoren zeigen jedoch eine schlechte Verträglichkeit in gewissen Substraten. Sie neigen stark zum Ausschwitzen, Sublimieren und/oder zum Verflüchtigen während der Verarbeitung zu Folien, Filmen, Fasern etc. bei hohen Temperaturen. Auf diese Weise geht ein unangemessen hoher Teil des Benzotriazolstabilisators verloren, insbesondere bei extrem dünnen Filmen und Ueberzügen. Dies gilt auch, wenn die Filme oder Ueberzüge während der Verwendung erhöhter Temperatur ausgesetzt sind.

Es sind Versuche unternommen worden, durch strukturelle Abwandlung des Benzotriazols die Verträglichkeit zu verbessern und den Stabilisatorschwund zu reduzieren.

Aus JP Kokai 158 588/75 sind o-Hydroxyphenyl-2H-benzotriazole bekannt, welche am Phenylring mit α,α-Dimethyl-benzyl substituiert sind.

US-PS 4 127 586 beschreibt o-Hydroxyphenyl-2H-benzotriazole, welche am Phenylring eine unsubstituierte Benzyl- oder α-Methylbenzylgruppe tragen können.

Ferner sind z.B. aus CS-PS 157 761 o-Hydroxyphenyl-2H-benzotriazole bekannt, welche am Phenylring eine gegebenenfalls substituierte 2-Hydroxybenzyl-Gruppe tragen.

Die erfindungsgemässen o-Hydroxyphenyl-2H-benzotriazole, welche am Phenylrest mindestens eine gegebenenfalls substituierte 4-Hydroxybenzyl-Gruppe tragen, zeigen eine ausgezeichnete Verträglichkeit und Löslichkeit in einer Vielzahl von Substraten und gleichzeitig einen überraschend geringe Flüchtigkeit in stabilisierten Zusammensetzungen während der Hochtemperatur-Verarbeitung oder bei deren Endverwendung. Die stabilisierten Mischungen, welche als Filme und Ueberzüge ständigen Witterungs- und Lichteinwirkungen ausgesetzt sind, werden wirksamer geschützt, als durch andere, bekannte 2-Aryl-2H-benzotriazole, welche strukturell den erfindungsgemässen Verbindungen nahestehen.

Die Erfindung betrifft Verbindungen der Formel I

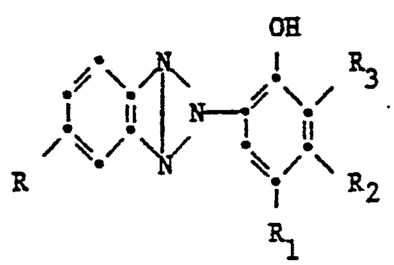

(I),

worin R Wasserstoff, Chlor, $C_1$-$C_4$ Alkyl oder $C_1$-$C_4$ Alkoxy ist, $R_2$ Wasserstoff, Hydroxy, $C_1$-$C_{12}$ Alkoxy oder Aralkoxy mit 1-5 C-Atomen in der Alkylkette bedeutet, $R_1$ und $R_3$ unabhängig voneinander Wasserstoff, Chlor, $C_1$-$C_{12}$ Alkyl, $C_3$-$C_4$ Alkenyl, Cycloalkyl, Phenyl, Aralkyl mit 1-5 C-Atomen in der Alkylkette oder die Gruppe der Formel II

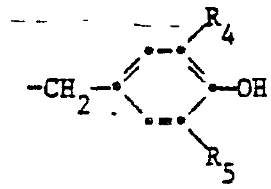

(II),

bedeuten, wobei mindestens eines von $R_1$ und $R_3$ die Gruppe der Formel II bedeutet und worin $R_4$ Wasserstoff, Chlor, $C_1$-$C_8$ Alkyl, Cycloalkyl, Phenyl oder Aralkyl mit 1-5 C-Atomen in der Alkylkette ist und $R_5$ für Wasserstoff, $C_1$-$C_8$ Alkyl, $C_1$-$C_{12}$ Alkoxy, Cycloalkyl, Phenyl, Aralkyl mit 1-5 C-Atomen in der Alkylkette oder für die Gruppe der Formel II steht, worin die Substituenten $R_4$ und $R_5$ die genannte Bedeutung haben, mit der Bedingung, dass $R_5$ nicht die Gruppe der Formel II bedeutet.

Bedeutet R $C_1$-$C_4$ Alkyl, so kann es sich dabei z.B. um Methyl, Aethyl, Isopropyl oder tert.-Butyl, insbesondere um Methyl handeln. Bedeutet R $C_1$-$C_4$ Alkoxy, so kann es z.B. Aethoxy, n-Butoxy oder insbesondere Methoxy sein.

Bevorzugt ist R Wasserstoff oder Chlor, insbesondere Wasserstoff.

Bedeutet $R_1$ oder $R_3$ $C_1$-$C_{12}$ Alkyl, so kann es sich z.B. um Methyl, Isopropyl, n-Propyl, sec.-Butyl, tert.-Butyl, tert.-Amyl, n-Hexyl, n-Octyl, tert.-Octyl, 2-Aethylhexyl oder n-Dodecyl handeln. Bevorzugt besitzten die Alkylgruppen $R_1$ und $R_3$ 1-8 C-Atome, und insbesondere 1-4 C-Atome.

Bedeutet $R_1$ oder $R_3$ $C_3$-$C_4$ Alkenyl, so kann es sich z.B. um Allyl oder Methallyl handeln. Bevorzugt ist Allyl.

Bedeutet $R_1$ oder $R_3$ Cycloalkyl, so kann es sich z.B. um Cyclopentyl, Cyclohexyl oder Cyclooctyl handeln. Bevorzugt ist Cyclohexyl.

Bedeutet $R_1$ oder $R_3$ Aralkyl mit 1-5 C-Atomen in der Alkylkette, so handelt es sich z.B. um Benzyl, α-Phenyläthyl, α,α-Dimethylbenzyl oder um 2-Phenyläthyl, bevorzugt um α-Phenyläthyl und α,α-Dimethylbenzyl.

Bevorzugt ist eines der Symbole $R_1$ und $R_3$ Wasserstoff, $C_1$-$C_{12}$ Alkyl oder die Gruppe der Formel II, während das andere jeweils die Gruppe der Formel II bedeutet.

$R_2$ ist als $C_1$-$C_{12}$ Alkoxy beispielsweise Methoxy, Aethoxy, Isopropoxy, n-Butoxy, n-Hexyloxy oder n-Dodecyloxy. Bevorzugt besitzt $R_2$ als Alkoxy 1-8 C-Atome, insbesondere 1-4 C-Atome.

$R_2$ besitzt als Aralkoxy 1-5 C-Atome in der Alkylkette und kann beispielsweise Benzyloxy sein. Aralkoxygruppen $R_2$ können gegebenenfalls am Aryl-Rest mit $C_1$-$C_4$ Alkyl substituiert sein und z.B. 4-tert.-Butylbenzyloxy bedeuten. Bevorzugt ist $R_2$ Benzyloxy.

Bevorzugt ist $R_2$ Wasserstoff, Hydroxy oder $C_1$-$C_{12}$ Alkoxy, insbesondere Wasserstoff.

$R_4$ und $R_5$ sind als $C_1$-$C_8$ Alkyl z.B. Methyl, Aethyl, n-Propyl, Isopropyl, sec.-Butyl, tert.-Butyl, n-Hexyl, tert.-Octyl oder n-Octyl. Bevorzugt sind $R_4$ und $R_5$ tert.-Butyl.

$R_4$ und $R_5$ sind als Cycloalkyl z.B. Cyclopentyl, Cyclohexyl oder Cyclooctyl, bevorzugt Cyclohexyl.

$R_4$ und $R_5$ können Aralkyl mit 1-5 C-Atomen in der Alkylkette bedeuten, wie z.B. Benzyl, α-Phenyläthyl oder α,α-Dimethylbenzyl. Bevorzugt sind $R_4$ und $R_5$ α-Phenyläthyl oder α,α-Dimethylbenzyl.

Bedeutet $R_5$ $C_1$-$C_{12}$ Alkoxy, so kann es beispielsweise Methoxy, Aethoxy, Isopropoxy, n-Butoxy, n-Hexyloxy oder n-Dodecyloxy sein. Bevorzugt besitzt $R_5$ als Alkoxy 1-8 C-Atome, insbesondere 1-4 C-Atome.

Bevorzugt sind $R_4$ und $R_5$ unabhängig voneinander $C_1$-$C_8$ Alkyl, besonders bevorzugt $C_1$-$C_4$ Alkyl.

Bevorzugt sind Verbindungen der Formel I, worin eines der Symbole $R_1$ und $R_3$ die Gruppe der Formel II bedeutet und das andere Wasserstoff, $C_1$-$C_8$ Alkyl, Cyclohexyl, Aralkyl mit 1-5 C-Atomen im Alkylrest oder die Gruppe der Formel II ist, worin $R_4$ und $R_5$ die zuvor genannte Bedeutung haben.

Spezielle bevorzugte erfindungsgemässe Verbindungen sind solche, welche den folgenden Formeln VI bis X entsprechen:

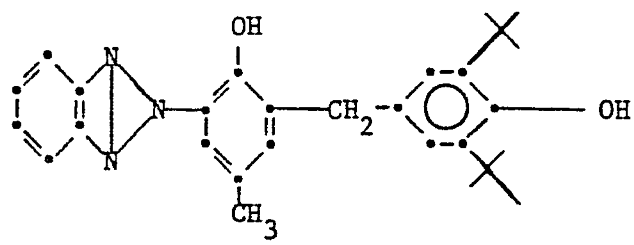
, (VI)

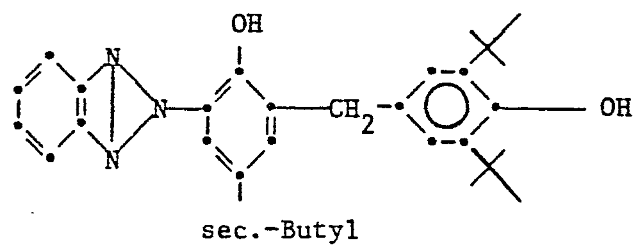
, (VII)

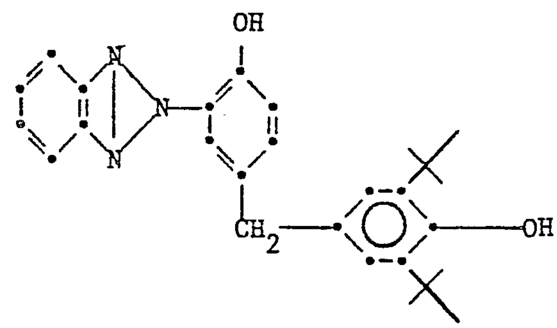
, (VIII)

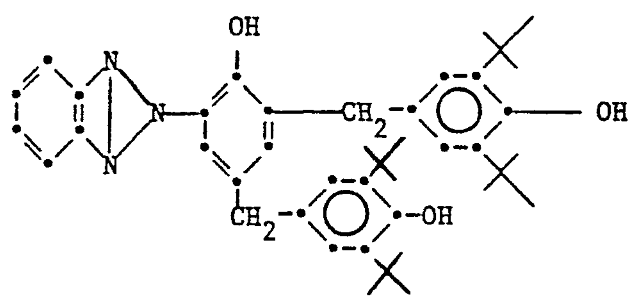
, (IX)

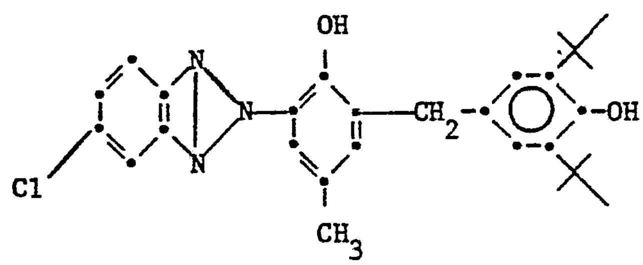

(X)

.

Die erfindungsgemässen Verbindungen können nach an sich bekannten Methoden hergestellt werden, z.B. indem man eine Verbindung der Formel III

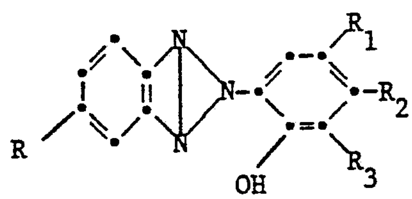

(III),

worin die Symbole R, $R_1$, $R_2$ und $R_3$ die bereits genannte Bedeutung haben, mit der Bedingung, dass von $R_1$ und $R_3$ mindestens eines der Symbole Wasserstoff bedeutet und dass $R_1$ und $R_3$ nicht die Gruppe der Formel II bedeuten können, mit einer Verbindung der Formel IV

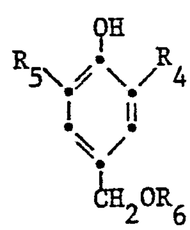

(IV),

worin $R_6$ Wasserstoff, $C_1$-$C_4$-Alkyl oder Acetyl ist und $R_4$ und $R_5$ die bereits genannte Bedeutung haben, mit der Bedingung, dass mindestens eines der Symbole $R_4$ und $R_5$ $C_1$-$C_8$ Alkyl ist, in Gegenwart einer starken, nicht oxidierenden Säure umsetzt, und so erhaltene Verbindungen der Formel I, worin mindestens eines von $R_4$ und $R_5$ Wasserstoff bedeutet, gegebenenfalls nochmals mit einer Verbindung der Formel IV, worin eines von $R_4$ und $R_5$ $C_1$-$C_8$-Alkyl ist und das andere Wasserstoff, Chlor, $C_1$-$C_8$-Alkyl, Cycloalkyl, Phenyl oder Aralkyl mit 1-5 C-Atomen in der Alkylkette darstellt, umsetzt.

Bedeutet $R_6$ $C_1$-$C_4$ Alkyl, so kann es sich dabei z.B. um Methyl, Aethyl, Isopropyl oder tert.-Butyl, insbesondere um Methyl handeln.

Bevorzugt ist $R_6$ Methyl.

Bevorzugt findet die Reaktion in einem inerten Lösungsmittel bei 30-80°C statt.

Geeignete inerte Lösungsmittel sind z.B. aliphatische und aromatische Kohlenwasserstoffe, sowie halogenierte Kohlenwasserstoffe, wie z.B. Methylenchlorid oder Toluol.

Als starke nicht oxidierende Säuren eignen sich z.B. Schwefelsäure, Phosphorsäure oder p-Toluolsulfonsäure. Ebenfalls geeignet sind stark saure anorganische und organische Ionenaustauscher bzw. Ionenaustausch-Harze, wie z.B. Montmorillonite oder sulfonierte Polystyrole. Besonders geeignet ist p-Toluolsulfonsäure.

Die Umsetzung des o-Hydroxyphenyl-2H-benzotriazols mit dem Phenol der Formel IV kann sowohl in stöchiometrischen Mengen als auch mit einem Ueberschuss der Verbindung der Formel IV in Gegenwart von einem Ionenaustauscher oder mindestens 0,01 Mol Säure erfolgen.

Die Umsetzung kann sowohl in einem homogenen System, wie z.B. Eisessig und Schwefelsäure oder Toluol und Toluolsulfonsäure, als auch im Zwei-Phasen-System durchgeführt werden, indem die Verbindungen der Formeln III und IV gelöst im inerten Lösungsmittel die eine Phase bilden und die wässrige, starke Säure (60-90%) die zweite Phase darstellt.

In diesem Fall beträgt die Reaktionstemperatur im allgemeinen -5° bis 50°C, vorzugsweise 0-25°C, besonders bevorzugt 5-15°C.

Die erfindungsgemässen Verbindungen können ebenfalls hergestellt werden, indem man eine Verbindung der Formel III mit einer Verbindung der Formel V

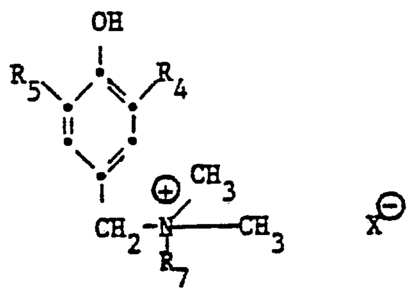

(V),

worin $R_4$ und $R_5$ die bereits genannte Bedeutung haben mit der Bedingung, dass mindestens eines der Symbole $R_4$ und $R_5$ $C_1$-$C_8$ Alkyl ist, und $R_7$ $C_1$-$C_3$ Alkyl, $C_3$-$C_4$ Alkenyl oder Aralkyl mit 1-5 C-Atomen in der Alkylkette ist und $X^{\ominus}$ Halogenid, Methylsulfat oder Dimethylphosphit bedeutet, in Gegenwart einer starken Base umsetzt, und so erhaltene Verbindungen der Formel I, worin mindestens eines von $R_4$ und $R_5$ Wasserstoff bedeutet, gegebenenfalls nochmals mit einer Verbindung der Formel V, worin eines von $R_4$ und $R_5$ $C_1$-$C_8$ Alkyl ist und das andere Wasserstoff, Chlor, $C_1$-$C_8$ Alkyl, Cycloalkyl, Phenyl oder Aralkyl mit 1-5 C-Atomen in der Alkylkette darstellt, umsetzt.

Bedeutet $R_7$ $C_1$-$C_3$ Alkyl, so kann es sich dabei z.B. um Methyl, Aethyl oder Isopropyl, insbesondere um Methyl handeln.

Bedeutet $R_7$ $C_3$-$C_4$ Alkenyl, so kann es Methallyl oder insbesondere Allyl sein.

$R_7$ kann Aralkyl mit 1-5 C-Atomen in der Alkylkette bedeuten, wie z.B. Benzyl, α-Phenyläthyl oder α,α-Dimethylbenzyl. Bevorzugt ist Benzyl.

Bevorzugt ist $R_7$ $C_1$-$C_3$ Alkyl.

$X^{\ominus}$ ist als Halogenid z.B. Chlorid, Bromid oder Jodid, bevorzugt Chlorid.

Bevorzugt ist $X^{\ominus}$ Methylsulfat.

Bevorzugt findet die Reaktion in einem polaren Lösungsmittel bei 80-160°C statt.

Geeignete polare Lösungsmittel sind z.B. Aethanol/Wasser-Gemische, Butanol, Dimethylformamid, Cellosclve, etc.

Als starke Basen eignen sich z.B. KOH, NaOH, Kalium-tert.-butylat, Natriummethylat oder Lithiumamid. Bevorzugt ist KOH oder NaOH.

Die Umsetzung des o-Hydroxyphenyl-2H-benzotriazols mit dem Phenol der Formel V kann sowohl in stöchiometrischen Mengen als auch mit einem Ueberschuss der Verbindung der Formel V in Gegenwart einer dem Phenol der Formel V äquivalenten Menge Base erfolgen.

Die erfindungsgemässen Verbindungen der Formel I können ebenfalls auf bekannte Weise hergestellt werden, in dem man zuerst das Phenol, das dem Benzotriazol zu Grunde liegt, mit dem Phenol der Formel IV oder der Formel V benzyliert und anschliessend über Kupplung und reduktive Triazolierung die gewünschte Verbindung der Formel I erhält.

Die erfindungsgemässen Verbindungen der Formel I, in denen nach der Umsetzung mindestens eines der Symbole $R_4$ und $R_5$ für $C_1$-$C_8$ Alkyl steht, können in an sich bekannter Weise entalkyliert werden, indem man die Verbindungen in Gegenwart der zuvor genannten Säuren auf 80-120°C erhitzt.

Eine teilweise Entalkylierung kann bereits bei der Umsetzung des o-Hydroxyphenyl-2H-benzotriazols mit dem Phenol der Formel IV oder Formel V stattfinden.

Die entstandenen entalkylierten Verbindungen der Formel I können anschliessend in an sich bekannter Weise mit den für $R_4$ bzw. $R_5$ genannten Substituenten, die von Wasserstoff verschieden sind, substituiert werden.

Die Ausgangsverbindungen der Formel III können nach dem in US-PS 4 041 044 beschriebenen Verfahren hergestellt werden. Die übrigen Ausgangsverbindungen sind im Handel erhältlich oder nach bekannten Methoden auf einfache Weise herzustellen.

Die erfindungsgemässen Verbindungen sind wirksame Lichtstabilisatoren für organische Materialien, so z.B. für eine grosse Anzahl Polymere. Im allgemeinen werden die erfindungsgemässen Stabilisatoren in einer Menge von 0,1-5 Gew.%, bevorzugt 0,5 bis 3 Gew.%, bezogen auf das organische Material, eingesetzt. Geeignete Polymere sind:

1. Polymere, welche von Mono- oder Diolefinen stammen, z.B. gegebenenfalls vernetztes Polyäthylen, Polypropylen, Polyisobutylen, Polymethylbuten-1, Polymethylpenten-1, Polyisopren, Polybuten-1 und Polybutadien.

2. Gemische der Homopolymere von 1), z.B. Gemische von Polypropylen und Polyäthylen, Polypropylen und Polyisobutylen, Polypropylen und Polybuten-1.

3. Copolymere aus den Monomeren für die Homopolymeren unter 1), z.B. Aethylen/Propylen-Copolymer, Propylen/Buten-1-Copolymer, Propylen/Isobutylen-Copolymer, Aethylen/Buten-1-Copolymer sowie Terpolymere von Aethylen und Propylen mit einem Dien, z.B. Hexadien, Dicyclopentadien oder Aethyliden-Norbornen, und Copolymere von α-Olefinen, z.B. Copolymere von Aethylen mit Acryl- oder Methacrylsäure.

4. Polystyrol.

5. Copolymere von Styrol und von α-Methylstyrol, z.B. Styrol/Butadien-Copolymer, Styrol/Acrylnitril-Copolymer, Styrol/Acrylnitril/Methacrylat-Copolymer, Styrol/Acrylnitril-Copolymer modifiziert mit Acrylester-Polymer, z.B. Styrol/Butadien/Styrol-Block-Copolymer.

6. Pfropf-copolymere von Styrol, z.B. das Pfropf-polymer von Styrol auf Polybutadien, das Propf-polymer von Styrol mit Acrylnitril auf Polybutadien, sowie ihre Gemische mit den Copolymeren von 5), bekannt als Acrylnitril/Butadien/Styrol oder ABS.

7. Halogenhaltige Vinyl-polymere, z.B. Polyvinylchlorid, Polyvinylidenchlorid, Polyvinylfluorid, Polychloropren, Vinylchlorid/Vinylidenchlorid-copolymer, Vinylchlorid/Vinylacetat-copolymer, Vinylidenchlorid/Vinylacetat-copolymer.

8. Polymere, welche von $\alpha,\beta$-ungesättigten Säuren stammen und ihre Derivate, Polyacrylate und Polymethacrylate, Polyacrylamide und Polyacrylnitril. Die erfindungsgemässen Verbindungen werden vorteilhafterweise in hitzehärtbaren Acrylharz-Lacken verwendet, welche aus einem Acrylharz und einem Melamin-Formaldehydharz bestehen.

9. Polymere, hergestellt aus ungesättigten Alkoholen und Aminen und von deren Acyl-derivativen oder Acetalen, z.B. Polyvinylalkohol, Polyvinylacetat, Polyvinylstearat, Polyvinylbenzoat, Polyvinylmaleat, Polyvinylbutyral, Polyallylphthalat, Polyallylmelamin und ihre Copolymere mit anderen Vinyl-Verbindungen z.B. Aethylen/Vinylacetat-copolymer.

10. Homopolymere und Copolymere hergestellt aus Epoxiden, z.B. Polyäthylenoxid, oder die Polymere der bis-Glycidyläther.

11. Polyacetale, z.B. Polyoxymethylen, sowie Polyoxymethylene, welche Aethylenoxid als Comonomer enthalten.

12. Polyalkylenoxide, z.B. Polyäthylenoxid, Polypropylenoxid oder Polyisobutylenoxid.

13. Polyphenylenoxide.

14. Polyurethane und Polyharnstoffe.

15. Polycarbonate.

16. Polysulfone.

17. Polyamide und Copolyamide, hergestellt aus Diaminen und Dicarbonsäuren und/oder aus Aminocarbonsäuren oder deren Lactamen, z.B. Polyamid 6, Polyamid 6,6, Polyamid 6,10, Polyamid 11, Polyamid 12, Poly-m-phenylen-isophthalamid.

18. Polyester aus Dicarbonsäuren und Dialkoholen und/oder aus Hydroxycarbonsäuren oder deren Lactonen, z.B. Polyäthylenglycolterephthalat, Poly-1,4-dimethylol-cyclohexanterephthalat.

19. Vernetzte Polymere aus Aldehyden einerseits und aus Phenolen, Harnstoffen oder Melamin andererseits, z.B. Phenol/Formaldehyd-, Harnstoff/Formaldehyd- und Melamin/Formaldehyd-Harze.

20. Alkyd-Harze, z.B. Glycerin/Phthalsäure-Harze und deren Gemische mit Melamin/Formaldehyd-Harzen.

21. Ungesättigte Polyester-Harze aus den Copolyestern von gesättigten und ungesättigten Dicarbonsäuren mit mehrwertigen Alkoholen sowie aus Vinyl-Verbindungen als Vernetzungsmittel und deren halogenhaltigen, flammresistenten Modifikationen.

22. Natürliche Polymere, z.B. Cellulose, Gummi, sowie deren chemisch modifizierte Derivative, z.B. Celluloseacetate, Cellulosepropionate und Cellulosebutyrate und die Celluloseäther, z.B. Methylcellulose.

Die erfindungsgemässen Verbindungen sind nicht nur wirksame Lichtschutzmittel, wie die 2-Aryl-2H-benzotriazole im allgemeinen, sondern dank ihres überraschend geringen Verlustes durch Verflüchtigung bei hoher Temperatur auch besonders wertvoll zum Stabilisieren von Polymeren, welche bei hohen Temperaturen verarbeitet werden müssen.

Die erfindungsgemässen Verbindungen werden daher vorzugsweise zum Stabilisieren von Polyestern wie z.B. Polyäthylenterephthalat, Polybutylenterephthalat oder deren Copolymere, von Polycarbonaten, z.B. aus Bisphenol A und Phosgen oder deren Copolymere, von Polysulfonen, Polyamiden, z.B. Nylon-6, Nylon-6,6, Nylon-6,10 etc.

sowie Copolyamide, von duroplastischen Acrylharzen, von thermoplastischen Acrylharzen, von Polyolefinen, z.B. Polyäthylen, Polypropylen, Copolyolefine etc., eingesetzt.

Die Stabilisatoren der Formel I können nach den herkömmlichen Methoden in das organische Material eingearbeitet werden, so z.B. in jeder beliebigen Phase während der Herstellung von geformten Produkten. Sie können zum Beispiel als Pulver, Suspensionen oder Emulsionen zum Polymer gemischt werden, welches als Pulver, Suspension oder Emulsion vorliegen kann.

Die stabilisierten Mischungen der Erfindung können gegebenenfalls 0,1 bis 5 Gew.%, vorzugsweise 0,5 bis 3 Gew.%, bezogen auf das Polymer, der üblichen Zusatzstoffe enthalten, insbesondere Antioxydantien, Lichtstabilisatoren, oder deren Gemische. Beispiele für solche Zusatzstoffe sind:

Antioxydantien, wie sterisch gehinderte Phenole; Lichtschutzmittel wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, Nickelverbindungen, sterisch gehinderte Amine und Oxalsäurediamide; Metalldesaktivatoren; Phosphite; peroxidzerstörende Verbindungen; Polyamidstabilisatoren; basische Co-Stabilisatoren; Nukleierungsmittel oder sonstige Zusätze, wie z.B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Gewisse hydrophobe, nicht diffundierende Hydroxyphenyl-2H-benzotriazole sind bekannt als UV-Absorber in photographischer Gelatine (US-PS 3 253 921 und US-PS 4 042 394). Die erfindungsgemässen Verbindungen sind wegen ihrer geringen Flüchtigkeit, ihrer guten Absorptionseigenschaften im UV-Bereich und ihrer photographischen Inaktivität besonders gut geeignet zum Stabilisieren von Farbbildern.

Von besonderem Interesse sind die duroplastischen und thermoplastischen Acrylharze, welche für Autolackierungen verwendet werden. Diese Stoffe sind in "Encyclopedia of Polymer Science and Technology", Interscience Publishers, New York, Band 1 (1964), auf Seiten 273-276 und Band 13 (1970), auf Seiten 530-532 beschrieben, ferner in "Understanding Paint" von W.R. Fuller, in American Paint Journal, St. Louis, 1965, Seite 124-135.

Acrylharzlacke, welche gemäss der Erfindung gegen die Einwirkung von Licht, Sauerstoff und Feuchtigkeit stabilisiert werden, sind die üblichen Einbrennlacke, wie z.B. in H. Kittel's "Lehrbuch der Lacke und Beschichtungen", Band 1, Teil 2, Seite 735 und 742 (Berlin, 1972), und in H. Wagner, H.F. Sarx, "Lackkunstharze", Seite 229-235 beschrieben.

Von besonderem Interesse ist die Stabilisierung von Metalleffektlacken auf der Basis von heissvernetzbarem Acrylharz mit den erfindungsgemässen Verbindungen. Aus diesen Harzen hergestellter, unstabilisierter Metalleffektlack ist trotz der ausgezeichneten physikalischen und chemischen Eigenschaften ungeeignet, da das UV-Licht die Deckschicht ungehindert passieren kann und in der unteren Schicht zur Rissbildung führt. Andere Lacke und heissvernetzbare Beschichtungen auf der Basis von Acrylharz, Melaminharz, Alkydharz, Polyesterharz, Epoxyharz oder deren Gemischen können ebenfalls mit den erfindungsgemässen Verbindungen wirksam stabilisiert werden.

Um die Metalleffektwirkung zu erzielen, verwendet man die üblichen Aluminium-Pigmente in 1-10 Gew.-%, bezogen auf das lösungsmittelfreie Bindemittel (Lackharz). Das Auftragen der stabilisierten Lacke kann nach den herkömmlichen Einschicht- oder Zweischicht-Verfahren geschehen. Im letzteren Falle wird der das Pigment enthaltende Vorlegelack zuerst aufgetragen und nachher mit Klarlack überzogen.

Weitere Zusatzstoffe, die im Lack enthalten sein können, sind andere übliche Lichtschutzmittel, phenolische Antioxydantien, Pigmente, Farbstoffe, Metalldesaktivatoren etc.

Ein typischer Lack gemäss der Erfindung enthält

a) heissvernetzbares Acrylharz und

b) 0,1 bis 5 Gew.-%, bezogen auf das Harz, einer Verbindung der Formel I, und

c) 0,1 bis 5 Gew.%, bezogen auf das Harz, eines sterisch gehinderten Amin-Stabilisators.

Bevorzugt enthält der Lack 0,5 bis 2 Gew.%, bezogen auf das Harz, von der Komponente b) und 0,5 bis 2 Gew.-%, bezogen auf das Harz, von der Komponente c).

Geeignete sterisch gehinderte Amine sind z.B. solche wie sie in der DE-OS 2 500 134, in der US 4 110 304 oder im "Taschenbuch der Kunststoff-Additive" von R.Gächter und R.Müller (Hauser Verlag München, 1979) beschrieben sind. Insbesondere geeignet sind Bis-(2,2,6,6-tetramethyl-4-piperidyl)-sebacat oder Bis-(1,2,2,6,6-pentamethyl-4-piperidyl)-2-n-butyl-2-(3,5-di-tert.butyl-4-hydroxybenzyl)malonat.

Die Kombination von sterisch gehinderten Aminen und den erfindungsgemässen Stabilisatoren ermöglicht sowohl eine ausgezeichnete Glanzerhaltung bei Bewitterung als auch Beständigkeit gegen das Abblättern metallisierter Einbrennlacke für Automobil-Decklackierungen.

Die erfindungsgemässen Benzotriazole unterscheiden sich in vier hervorragenden Eigenschaften von den strukturell nahestehenden Benzotriazolen des Standes der Technik. Sie sind:

1. schwerflüchtig

2. gut löslich in den Lösungsmitteln, welche für Kunststoff-Überzüge üblicherweise verwendet werden.

3. besser verträglich mit Polyolefinen oder Vinylpolymeren.

4. widerstandfähig gegenüber Metallionen, welche häufig in Stabilisator- und Härtersystemen für Polymere anzutreffen sind. Viele bekannte Benzotriazol-Stabilisatoren bilden offensichtlich farbige Metallkomplexe von unbekannter Struktur mit Ionen, wie Kobalt, Eisen, Barium, Cadmium, Zinn etc. Solche Metallionen kommen zum Beispiel in Polymeren, wie z.B. in ungesättigten Polyestern, Alkyden etc. vor, wenn man Kobaltnaphtenat zum Trocknen verwendet; in PVC oder PVC-Copolymeren, welche mit Barium/Cadmium oder mit Zinn-verbindungen stabilisiert sind; in Polyurethanen, die Zinn-Katalysatoren enthalten; in ABS-Harzen, welche mit Eisen verunreinigt sind. In all diesen Substraten können die erfindungsgemässen Benzotriazole verwendet werden, dank ihrer Eigenschaft, mit den erwähnten Metallionen keine Komplexe zu bilden.

Die Schwerflüchtigkeit, insbesondere wenn mit guter Substratverträglichkeit und Löslichkeit kombiniert, erlaubt die unproblematische Einarbeitung der erfindungsgemässen Benzotriazole in das Polymer, wo sie auch nach der Hochtemperatur-Verarbeitung verbleiben und daher dem Endprodukt die gewünschte Stabilität verleihen.

Die grössere Substratverträglichkeit der erfindungsgemässen Verbindungen zusammen mit der bereits diskutierten Schwerflüchtigkeit gestattet die Verarbeitung und Verwendung der stabilisierten Produkte bei höheren Temperaturen, sowie eine verlängerte Gebrauchsdauer der Produkte bei üblichen Temperaturen. Dadurch wird das unerwünschte Ausschwitzen des Stabilisators während der Verarbeitung verhindert, was sonst häufig zu kostspieligen Schädigungen der Produktionsanlage führt.

Die folgenden Beispiele erläutern die Erfindung. Prozente sind Gewichtsprozente.

Beispiel 1: 225,3 g (1 Mol) 2-(2-Hydroxy-5-methylphenyl)-benztriazol werden mit 250,4 g (1 Mol) 2,6-Di-tert.-butyl-4-methoxymethylphenol in 1800 ml Methylenchlorid gelöst und die erhaltene Lösung wird auf 10°C gekühlt. Bei dieser Temperatur tropft man in ca. 2 Stunden unter starkem Rühren 460 g 80%ige Schwefelsäure hinzu. Das Reaktionsgemisch wird anschliessend 12 Stunden bei Raumtemperatur ausgerührt, die Schwefelsäure abgetrennt, die Methylenchloridlösung mit Wasser säurefrei gewaschen, über Natriumsulfat getrocknet und eingedampft. Der Rückstand wird aus Hexan umkristallisiert. Man erhält das 2-[2-Hydroxy-3-(3,5-di-tert.-butyl-4-hydroxybenzyl)-5-methylphenyl]-benztriazol (Verbindung 1) vom Smp. 148°C als fast farblose Kristalle.

Verwendet man anstelle von 2-(2-Hydroxy-5-methylphenyl)-benztriazol eine äquimolare Menge
2-(2-Hydroxy-5-phenylphenyl)-benztriazol bzw.
2-(2-Hydroxy-5-tert.-butylphenyl)-benztriazol bzw.
2-(2-Hydroxy-5-sec.-butylphenyl)-benztriazol bzw.
2-(2-Hydroxy-5-α,α-dimethylbenzylphenyl)-benztriazol
und verfährt man im übrigen wie oben beschrieben, so erhält man das
2-[2-Hydroxy-3-(3,5-di-tert.-butyl-4-hydroxybenzyl)-5-phenylphenyl]-benztriazol (Verbindung 2) vom Smp. 180°C bzw. das
2-[2-Hydroxy-3-(3,5-di-tert.-butyl-4-hydroxybenzyl)-5-tert.-butylphenyl]-benztriazol (Verbindung 3) vom Smp. 199-200° C bzw. das
2-[2-Hydroxy-3-(3,5-di-tert.-butyl-4-hydroxybenzyl)-5-sec.-butylphenyl]-benztriazol (Verbindung 4) vom Smp. 163-164°C bzw. das
2-[2-Hydroxy-3-(3,5-di-tert.-butyl-4-hydroxybenzyl)-5-α,α-dimethylbenzylphenyl]-benztriazol (Verbindung 5) vom Smp. 174-175°C.

Beispiel 2: 21,1 g (0,1 Mol) 2-(2-Hydroxyphenyl)-benztriazol und 25,0 g (0,1 Mol) 2,6-Di-tert.-butyl-4-methoxymethylphenol werden in 300 ml Methylenchlorid gelöst und die erhaltene Lösung wird auf 5°C abgekühlt. Bei dieser Temperatur tropft man in ca. 1 Stunde unter kräftigem Rühren 90 g 80%ige Schwefelsäure hinzu. Anschliessend wird 6 Stunden

bei Raumtemperatur ausgerührt, die Schwefelsäure abgetrennt, die Methylenchloridlösung säurefrei gewaschen, über Natriumsulfat getrocknet und eingedampft. Durch Kristallisation des erhaltenen Rückstandes aus Hexan erhält man das 2-[2-Hydroxy-5-(3,5-di-tert.-butyl-4-hydroxybenzyl)-phenyl]-benztriazol (Verbindung 6) als praktisch farblose Kristalle vom Smp. 150°C.

Verwendet man 50,0 g (0,2 Mol) 2,6-Di-tert.-butyl-4-methoxymethylphenol und lässt man bei sonst gleichen Reaktionsbedingungen die Temperatur beim Ausrühren auf 40-45°C steigen, so erhält man das 2-[2-Hydroxy-3,5-bis(3,5-di-tert.-butyl-4-hydroxybenzyl)-phenyl]-benztriazol (Verbindung 7) vom Smp. 170-171°C.

Beispiel 3: 12,0 g (0,05 Mol) 2-(2-Hydroxy-4-methoxyphenyl)-benztriazol und 12,5 g 2,6-Di-tert.-butyl-4-methoxymethylphenol werden wie in Beispiel 1 beschrieben umgesetzt. Man erhält nach Kristallisation des Rohproduktes aus Ligroin das 2-[2-Hydroxy-4-methoxy-5-(3,5-di-tert.-butyl-4-hydroxybenzyl)-phenyl]-benztriazol (Verbindung 8) vom Smp. 188-189°C.

Beispiel 4: 22,7 g (0,1 Mol) 2-(2,4-Dihydroxyphenyl)-benztriazol und 50,0 g (0,2 Mol) 2,6-Di-tert.-butyl-4-methoxymethylphenol werden wie in Beispiel 1 beschrieben umgesetzt. Nach Kristallisation des Rohproduktes aus Ligroin erhält man das 2-[2,4-Dihydroxy-3,5-bis(3,5-di-tert.-butyl-4-hydroxybenzyl)-phenyl]-benztriazol (Verbindung 9) vom Smp. 240°C.

Beispiel 5: 22,5 g (0,1 Mol) 2-(2-Hydroxy-5-methylphenyl)-benztriazol und 27,5 g (0,1 Mol) 2,6-Di-sec.-butyl-4-methoxymethylphenol werden wie in Beispiel 1 beschrieben umgesetzt. Nach Kristallisation aus Hexan erhält man das 2-[2-Hydroxy-3-(3,5-di-sec.-butyl-4-hydroxybenzyl)-5-methylphenyl]-benztriazol (Verbindung 10) vom Smp. 110-111°C.

Beispiel 6: 50,0 g 2-[2-Hydroxy-3-(3,5-di-tert.-butyl-4-hydroxybenzyl)-5-methylphenyl]-benztriazol (Verbindung 1) werden in 500 ml Xylol in Gegenwart von 2,5 g p-Toluolsulfonsäure 5 Stunden am Rückfluss (ca. 140°C) gekocht. Nach dem Abkühlen wird die Reaktionslösung mit Wasser säurefrei gewaschen, über Natriumsulfat getrocknet und eingedampft. Durch Kristallisation des erhaltenen Rückstandes aus Ligroin erhält man das 2-[2-Hydroxy-3-(3-tert.-butyl-4-hydroxybenzyl)-5-methylphenyl]-benztriazol (Verbindung 11) vom Smp. 149-150°C.

Verwendet man beim gleichen Ansatz 500 ml o-Dichlorbenzol anstelle von Xylol und erwärmt man das Reaktionsgemisch 6 Stunden auf 155-160° C, so erhält man nach gleicher Aufarbeitung das 2-[2-Hydroxy-3-(4-hydroxybenzyl)-5-methylphenyl]-benztriazol (Verbindung 12) vom Smp. 206-207°C.

Beispiel 7: 38,7 g (0,1 Mol) 2-[2-Hydroxy-3-(3-tert.-butyl-4-hydroxybenzyl)-5-methylphenyl]-benztriazol (Verbindung 11) und 25,0 g (0,1 Mol) 2,6-Di-tert.-butyl-4-methoxymethylphenol werden wie in Beispiel 1 beschrieben umgesetzt. Nach Kristallisation des Rohproduktes aus Ligroin erhält man das 2-{2-Hydroxy-3-[3-tert.-butyl-4-hydroxy-5-(3,5-di-tert.-butyl-4-hydroxybenzyl)-benzyl]-5-methylphenyl}-benztriazol (Verbindung 13) mit Smp. 88°C.

Aus der Ligroinmutterlauge kann nach Eindampfen und Chromatographie an Silicagel (Toluol/Hexan 1:1) das 2-{2-Hydroxy-3-[3-tert.-butyl-4-hydroxy-5-(3-tert.-butyl-4-hydroxybenzyl)-benzyl]-5-methylphenyl}-benztriazol (Verbindung 14) mit Smp. 144-145°C isoliert werden.

Beispiel 8: 69 g (0,5 Mol) o-Nitranilin werden diazotiert und die erhaltene Diazoniumsalzlösung wird zu einer Lösung von 156 g (0,5 Mol) 4,4'-Hydroxy-3',5'-di-tert.-butyl-diphenylmethan in 500 ml Wasser und 20 g Natriumhydroxyd bei ca. 5°C getropft. Während der Kupplung wird durch Zutropfen von 2n Natronlauge ein pH von 9-10 aufrechterhalten.

Nach beendeter Kupplung wird mit Essigsäure angesäuert und der erhaltene Azofarbstoff abgenutscht, mit Wasser gewaschen und getrocknet.

92,3 g (0,2 Mol) dieses Azofarbstoffes werden in 300 ml Aethylenglykolmonomethyläther und 100 ml 10%iger Natronlauge gelöst. Man gibt 50 g Zinkstaub hinzu und tropft dann innerhalb von 2 Stunden 100 ml 30%ige Natronlauge zum Reaktionsgemisch, wobei die Temperatur auf 45-50°C gehalten wird. Nach vollständiger Entfärbung des Reaktionsgemisches erwärmt man 20 Minuten am Rückfluss, filtriert vom Zinkschlamm ab und säuert das Filtrat mit verdünnter Salzsäure an. Der entstandene Niederschlag wird abgenutscht, mit Wasser gewaschen, getrocknet und aus Ligroin umkristallisiert. Man erhält das 2-[2-Hydroxy-5-(3,5-di-tert.-butyl-4-hydroxybenzyl)-phenyl]-benztriazol (Verbindung 6) vom Smp. 150°C.

Beispiel 9: 24,1 g (0,09 Mol) 2-(2-Hydroxy-5-tert.-butylphenyl)-benztriazol werden mit 38,9 g (0,1 Mol) Trimethyl-(4-hydroxy-3,5-di-tert.-butyl)-benzylammonium-methylsulfat in 250 ml n-Butanol gelöst. Nach Zugabe von 6,1 g (0,11 Mol) pulverisiertem Kaliumhydroxid wird das Reaktionsgemisch während 20 Stunden unter Rückfluss erhitzt. Nach dem Abkühlen wird das Gemisch mit 2n HCl-Lösung angesäuert und das entstandene Produkt mit Methylenchlorid extrahiert und eingedampft. Das rötliche Harz kristallisiert aus Methanol. Nach der Umkristallisation der schwach gelben Kristalle aus Ligroin erhält man ein weisses Kristallisat von 2-[2-Hydroxy-3-(3,5-di-tert.-butyl-4-hydroxybenzyl)-5-tert.-butylphenyl]-benztriazol. Smp. 199-200°C (Verbindung 3).

Verwendet man anstelle von 2-(2-Hydroxy-5-tert.-butylphenyl)-benztriazol eine äquimolare Menge 2-(2-Hydroxy-5-methylphenyl)-benztriazol und verfährt man im übrigen wie im Beispiel 9 beschrieben, so erhält man das 2-[2-Hydroxy-3-(3,5-di-tert.-butyl-4-hydroxybenzyl)-5-methylphenyl]-benztriazol. Smp. 148°C (Verbindung 1).

Beispiel 10: 24,5 g (0,1 Mol) 2-(2-Hydroxyphenyl)-5-chlorbenztriazol werden mit 26,0 g (0,11 Mol) 3,5-Di-tert.-butyl-4-hydroxy-benzylalkohol in 400 ml Eisessig suspendiert. Dazu tropft man bei 20-25°C in ca. 30 Minuten 20,0 g konz. Schwefelsäure. Das Reaktionsgemisch wird anschliessend 6 Stunden bei 20-25°C weitergerührt und dann in 2 Liter Eiswasser gegossen. Der entstandene Niederschlag wird abgenutscht, mit Wasser säurefrei gewaschen, getrocknet und aus Methanol umkristallisiert. Man erhält das 2-[2-Hydroxy-5-(3,5-di-tert.-butyl-4-hydroxybenzyl)-phenyl]-5-chlorbenztriazol (Verbindung 15) als farblose Substanz vom Smp. 140-141°C.

Verwendet man anstelle von 2-(2-Hydroxyphenyl)-5-chlorbenztriazol eine äquimolare Menge 2-(2-Hydroxy-3-tert.-butylphenyl)-benztriazol bzw. 2-(2-Hydroxy-5-methylphenyl)-5-chlorbenztriazol und verfährt man im übrigen wie im Beispiel 10 beschrieben, so erhält man das 2-[2-Hydroxy-3-tert.-butyl-5-(3,5-di-tert.-butyl-4-hydroxybenzyl)-phenyl]-benztriazol (Verbindung 16) vom Smp. 150-151°C bzw. das 2-[2-Hydroxy-3-(3,5-di-tert.-butyl-4-hydroxybenzyl)-5-methylphenyl]-5-chlorbenztriazol (Verbindung 17) vom Smp. 160°C.

Beispiel 11: 23,2 g (0,05 Mol) 2-[2-Hydroxy-5-(3,5-di-tert.-butyl-4-hydroxybenzyl)-phenyl]-5-chlorbenztriazol (Verbindung 15) werden in 200 ml Methylenchlorid gelöst. Dazu gibt man 60 g 80%ige Schwefelsäure und lässt unter kräftigem Rühren bei 35-40°C in ca. 5 Stunden eine Lösung von 13,0 g (0,055 Mol) 3,5-Di-tert.-butyl-4-hydroxybenzylalkohol in 80 ml Methylenchlorid zutropfen. Anschliessend wird noch 3 Stunden bei 35-40°C ausgerührt und dann nach Beispiel 1 aufgearbeitet. Durch Kristallisation des Eindampfrückstandes aus Methanol erhält man das 2-[2-Hydroxy-3,5-bis-(3,5-di-tert.-butyl-4-hydroxybenzyl)-phenyl]-5-chlorbenztriazol (Verbindung 18) vom Smp. 216-217°C.

Patentansprüche:

1. Verbindungen der Formel I

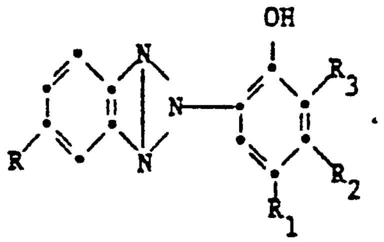

(I),

worin R Wasserstoff, Chlor, $C_1$-$C_4$ Alkyl oder $C_1$-$C_4$ Alkoxy ist, $R_2$ Wasserstoff, Hydroxy, $C_1$-$C_{12}$ Alkoxy oder Aralkoxy mit 1-5 C-Atomen in der Alkylkette bedeutet, $R_1$ und $R_3$ unabhängig voneinander Wasserstoff, Chlor, $C_1$-$C_{12}$ Alkyl, $C_3$-$C_4$ Alkenyl, Cycloalkyl, Phenyl, Aralkyl mit 1-5 C-Atomen in der Alkylkette oder die Gruppe der Formel II

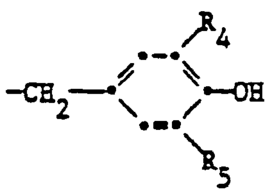

(II),

bedeuten, wobei mindestens eines von $R_1$ und $R_3$ die Gruppe der Formel II bedeutet und worin $R_4$ Wasserstoff, Chlor, $C_1$-$C_8$ Alkyl, Cycloalkyl, Phenyl oder Aralkyl mit 1-5 C-Atomen in der Alkylkette ist und $R_5$ für Wasserstoff, $C_1$-$C_8$ Alkyl, $C_1$-$C_{12}$ Alkoxy, Cycloalkyl, Phenyl, Aralkyl mit 1-5 C-Atomen in der Alkylkette oder für die Gruppe der Formel II steht, worin die Substituenten $R_4$ und $R_5$ die genannte Bedeutung haben, mit der Bedingung, dass $R_5$ nicht die Gruppe der Formel II bedeutet.

2. Verbindungen der Formel I gemäss Anspruch 1,

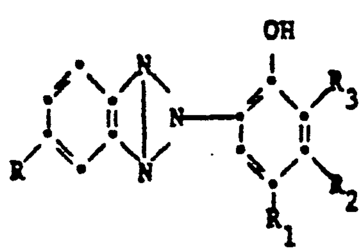

(I),

worin R Wasserstoff, Chlor, $C_1$-$C_4$ Alkyl oder $C_1$-$C_4$ Alkoxy ist, $R_2$ Wasserstoff, Hydroxy, $C_1$-$C_{12}$ Alkoxy oder Aralkoxy mit 1-5 C-Atomen in der Alkylkette bedeutet, $R_1$ und $R_3$ unabhängig voneinander Wasserstoff, Chlor, $C_1$-$C_{12}$ Alkyl, Cycloalkyl, Phenyl, Aralkyl mit 1-5 C-Atomen in der Alkylkette oder die Gruppe der Formel II

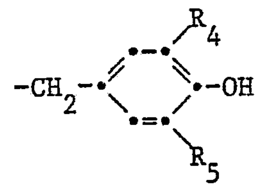

(II),

bedeuten, wobei mindestens eines von $R_1$ und $R_3$ die Gruppe der Formel II bedeutet und worin $R_4$ Wasserstoff, Chlor, $C_1$-$C_8$ Alkyl, Cycloalkyl, Phenyl oder Aralkyl mit 1-5 C-Atomen in der Alkylkette ist und $R_5$ für Wasserstoff, $C_1$-$C_8$ Alkyl, $C_1$-$C_{12}$ Alkoxy, Cycloalkyl, Phenyl, Aralkyl mit 1-5 C-Atomen in der Alkylkette oder für die Gruppe der Formel II steht, worin die Substituenten $R_4$ und $R_5$ die genannte Bedeutung haben, mit der Bedingung, dass $R_5$ nicht die Gruppe der Formel II bedeutet.

3. Verbindungen der Formel I gemäss Anspruch 1, dadurch gekennzeichnet, dass eines der Symbole $R_1$ und $R_3$ die Gruppe der Formel II bedeutet und das andere Wasserstoff, $C_1$-$C_8$ Alkyl, Cyclohexyl, Aralkyl mit 1-5 C-Atomen im Alkylrest oder die Gruppe der Formel II ist worin $R_4$ und $R_5$ die in Anspruch 1 genannte Bedeutung haben.

4. Verbindungen der Formel I gemäss Anspruch 1, dadurch gekennzeichnet, dass eines der Symbole $R_1$ und $R_3$ Wasserstoff, $C_1$-$C_{12}$ Alkyl oder die Gruppe der Formel II bedeutet, während das andere jeweils die Gruppe der Formel II bedeutet, worin $R_4$ und $R_5$ die in Anspruch 1 genannte Bedeutung haben.

5. Verbindungen der Formel I gemäss Anspruch 1, dadurch gekennzeichnet, dass $R_4$ und $R_5$ unabhängig voneinander $C_1$-$C_4$ Alkyl bedeuten.

6. Verbindungen der Formel I gemäss Anspruch 1, dadurch gekennzeichnet, dass R für Wasserstoff oder Chlor steht und $R_2$ Wasserstoff, Hydroxy oder $C_1$-$C_{12}$ Alkoxy ist.

7. Verbindung der Formel I gemäss Anspruch 1, dadurch gekennzeichnet, dass sie der Formel VI

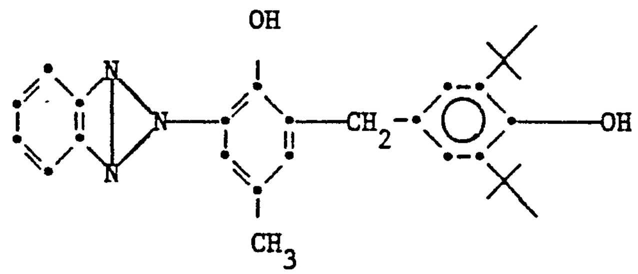

(VI)

oder der Formel VII

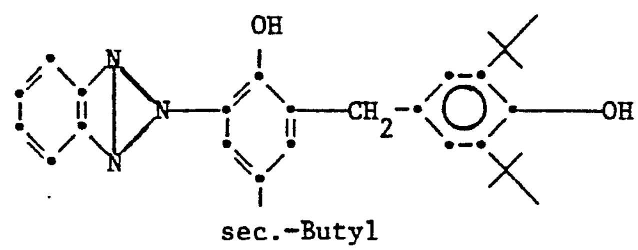

(VII)

oder der Formel VIII

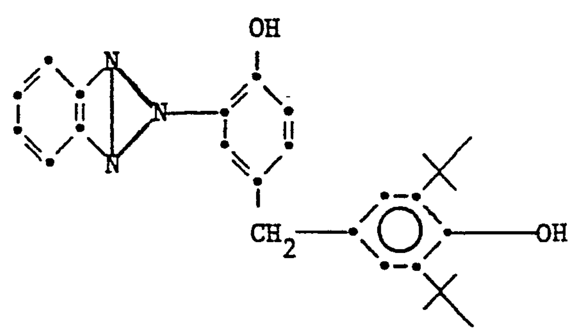

(VIII)

oder der Formel IX

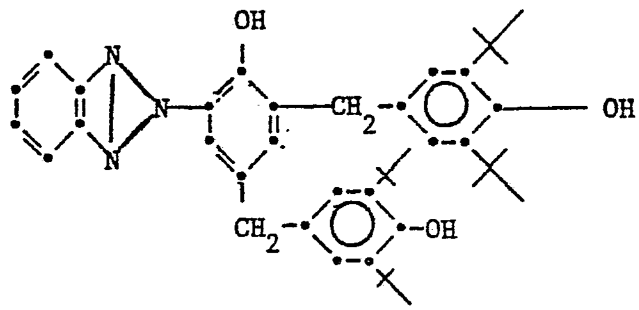

(IX)

oder der Formel X

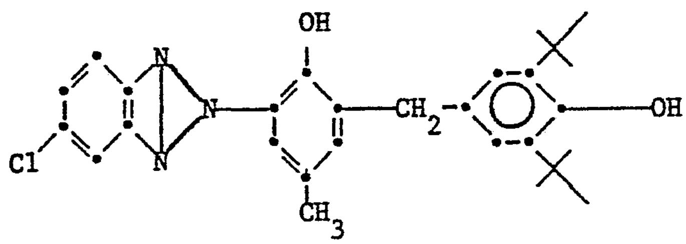

(X)

entspricht.

8. Stabilisiertes organisches Material enthaltend 0,1 - 5 Gew.-% eines Stabilisators gemäss Anspruch 1.

9. Stabilisiertes organisches Material gemäss Anspruch 8, dadurch gekennzeichnet, dass das Material ein Polymer ist.

10. Stabilisiertes Polymer gemäss Anspruch 9, dadurch gekennzeichnet, dass das Polymer ein Polyester, Polycarbonat, Polysulfon, Acrylharz, Polyamid oder ein Polyolefin ist.

11. Verfahren zur Herstellung von Verbindungen der Formel I gemäss Anspruch 1, dadurch gekennzeichnet, dass man eine Verbindung der Formel III

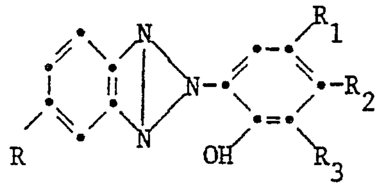

(III),

worin die Symbole R, $R_1$, $R_2$ und $R_3$ die in Anspruch 1 genannte Bedeutung haben, mit der Bedingung, dass von $R_1$ und $R_3$ mindestens eines der Symbole Wasserstoff bedeutet und dass $R_1$ und $R_3$ nicht die Gruppe der Formel II bedeuten können, mit einer Verbindung der Formel IV

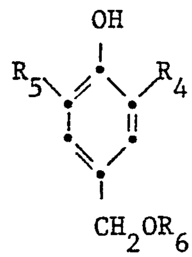

(IV),

worin $R_6$ Wasserstoff, $C_1$-$C_4$ Alkyl oder Acetyl ist und $R_4$ und $R_5$ die in Anspruch 1 genannte Bedeutung haben, mit der Bedingung dass mindestens eines der Symbole $R_4$ und $R_5$ $C_1$-$C_8$ Alkyl ist, in Gegenwart einer starken, nicht oxidierenden Säure umsetzt, und so erhaltene Verbindungen der Formel I, worin mindestens eines von $R_4$ und $R_5$ Wasserstoff bedeutet, gegebenenfalls nochmals mit einer Verbindung der Formel IV, worin eines von $R_4$ und $R_5$ $C_1$-$C_8$ Alkyl ist und das andere Wasserstoff, Chlor, $C_1$-$C_8$ Alkyl, Cycloalkyl, Phenyl oder Aralkyl mit 1-5 C-Atomen in der Alkylkette darstellt, umsetzt.

12. Verfahren zur Herstellung von Verbindungen der Formel I gemäss Anspruch 1, dadurch gekennzeichnet, dass man eine Verbindung der Formel III

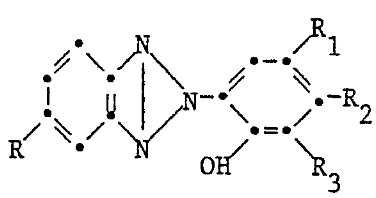

(III),

worin die Symbole R, $R_1$, $R_2$ und $R_3$ die in Anspruch 1 genannte Bedeutung haben, mit der Bedingung, dass von $R_1$ und $R_3$ mindestens eines der Symbole Wasserstoff bedeutet und dass $R_1$ und $R_3$ nicht die Gruppe der Formel II bedeuten können, mit einer Verbindung der Formel V

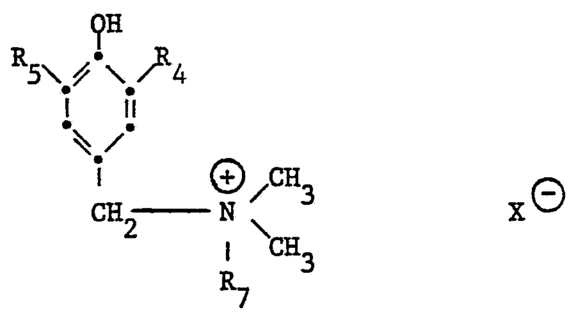

(V),

worin $R_4$ und $R_5$ die in Anspruch 1 genannte Bedeutung haben, mit der Bedingung, dass mindestens eines der Symbole $R_4$ und $R_5$ $C_1$-$C_8$ Alkyl ist, $R_7$ $C_1$-$C_3$ Alkyl, $C_3$-$C_4$ Alkenyl oder Aralkyl mit 1-5 C-Atomen in der Alkylkette ist und $X^{\ominus}$ Halogenid, Methylsulfat oder Dimethylphosphit bedeutet, in Gegenwart einer starken Base umsetzt, und so erhaltene Verbindungen der Formel I, worin mindestens eines von $R_4$ und $R_5$ Wasserstoff bedeutet, gegebenenfalls nochmals mit einer Verbindung der Formel V, worin eines von $R_4$ und $R_5$ $C_1$-$C_8$ Alkyl ist und das andere Wasserstoff, Chlor, $C_1$-$C_8$ Alkyl, Cycloalkyl, Phenyl oder Aralkyl mit 1-5 C-Atomen in der Alkylkette darstellt, umsetzt.